# EUROPEAN PATENT APPLICATION

(11) **EP 2 716 626 A1**
(43) Date of publication of application: **09.04.2014**
(21) Application number: 12792773.9
(22) Date of filing: 23.05.2012
(51) Int. Cl.: C07C 233/31, A61K 31/165, A61P 35/02, C07C 237/42

(54) **BENZYLAMINE DERIVATIVE**

(30) Priority: 27.05.2011 JP 2011119668
(71) Applicant: The University of Tokushima, Tokushima-shi Tokushima 770-8501 (JP)
(72) Inventor: TSUJI, Daisuke, Tokushima-shi Tokushima 770-8505 (JP); ITOH, Kohji, Tokushima-shi Tokushima 770-8505 (JP); OTAKA, Akira, Tokushima-shi Tokushima 770-8505 (JP); SHIGENAGA, Akira, Tokushima-shi Tokushima 770-8505 (JP); ABE, Masahiro, Tokushima-shi Tokushima 770-8503 (JP); HIASA, Masahiro, Tokushima-shi Tokushima 770-8504 (JP)
(74) Representative: Müller-Boré & Partner Patentanwälte PartG mbB
(86) International application number: PCT/JP2012/063213
(87) International publication number: WO 2012/165262

(57) **Abstract**

The objective of the present invention is to provide a new compound which is effective in treating blood cancer, particularly multiple myeloma, and which is also effective in suppressing an SP cell, i.e. Side Population Cell, which is a cause of recurrence of cancer. The compound according to the present invention is a novel compound which has the specific benzylamine structure.

## Description

### TECHNICAL FIELD

The present invention relates to a novel benzylamine derivative which is useful for treating blood cancer, a drug containing the benzylamine derivative, a blood cancer drug containing the benzylamine derivative, use of the benzylamine derivative for treating blood cancer, and a method for treating blood cancer using the benzylamine derivative.

### BACKGROUND ART

Blood cancer is a disease that involves a cancerous change of a cell in the blood, and is classified in terms of the kind of cancerous cell. Blood cancer is mainly classified into three diseases: leukemia, in which a hematopoietic stem cell in bone marrow becomes cancerous; malignant lymphoma, in which a lymphocyte becomes cancerous; and multiple myeloma, in which a plasma cell in bone marrow becomes cancerous.

It was once thought that blood cancer is incurable. However, the cure rate thereof has been increased due to medical advances. In particular, it has been possible that an early adequate treatment provides long-term survival for patients of leukemia and malignant lymphoma. However, new anticancer drug has been constantly required due to problems of drug resistance and the like.

With respect to multiple myeloma among blood cancer, cure is difficult, prognosis may not be good in many cases, and recurrence rate is high. In general, a therapy with both melphalan and prednisolone or a therapy with both cyclophosphamide and prednisolone is used as chemotherapy. The former therapy is called as "MP" and the latter therapy is called as "CP". However, since multiple myeloma is difficult to cure, new drug such as thalidomide

has been developed.

Various compounds have been developed as a candidate for development of an anticancer compound so far. For example, the compound described in Patent Document 1 is exemplified as an anticancer compound having a benzylamine structure.

### PRIOR ART

### PATENT DOCUMENT

Patent Document 1: JP 2007-515413 T

### DISCLOSURE OF THE INVENTION

### PROBLEMS TO BE SOLVED BY THE INVENTION

As described above, various compounds have been developed as an anticancer compound so far. However, since multiple myeloma among blood cancer is very difficult to cure, a more useful curative compound is required. In addition, even if symptom is once suppressed, cancer is always accompanied with a problem of recurrence. Therefore, a compound which is useful for suppression of recurrence is needed.

Under the above-described circumstance, the objective of the present invention is to provide a new compound which is effective in treating blood cancer, particularly multiple myeloma, and which is also effective in suppressing an SP cell, i.e. Side Population Cell, which is a cause of recurrence of cancer.

### MEANS FOR SOLVING THE PROBLEMS

The inventors of the present invention intensively studied for solving the above problem. As a result, the present inventors completed the present invention by finding that a novel compound which has the specific benzylamine structure exhibits selective cytotoxicity to a blood cancer cell and is particularly effective against an SP cell.

The benzylamine derivative of the present invention is characterized in being represented by the following formula (I) : 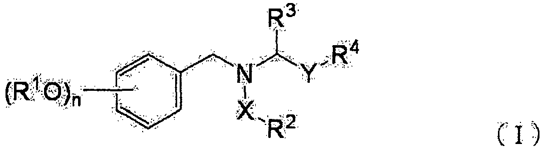 wherein
X is a carbonyl group: -C(=O)-, an oxycarbonyl group: -C(=O)O-, a sulfoxide group: -S(=O)- or a sulfonyl group: -S(=O)₂-;
Y is a carbonyl group or -CH(OR⁵)-;
R¹ is a hydrogen atom, a C₁₋₆ alkyl group or a benzyl group;
R² is a C₁₋₆ alkyl group, a C₂₋₆ alkenyl group, a C₂₋₆ alkynyl group or a phenyl group optionally having a substituent α;
R³ is a C₁₋₆ alkyl group optionally having a substituent β;
R⁴ is a hydrogen atom, an amino group, a hydroxyamino group, a C₁₋₆ alkoxyamino group, a carboxy group or a (C₁₋₆ alkoxy) carbonyl group;
R⁵ is a hydrogen atom, a C₁₋₇ alkanoyl group or a tri (C₁₋₆ alkyl)silyl group;
substituent α is one or more substituents selected from the group consisting of a C₁₋₆ alkyl group, a C₁₋₆ alkoxy group, a halogen atom, a cyano group and a nitro group;
substituent β is one or more substituents selected from the group consisting of a halogen atom, a hydroxy group, a C₁₋₆ alkoxy group and a benzyloxy group;
n is an integer of not less than 1 and not more than 5.

In the present invention, the term "C₁₋₆ alkyl group" is a linear or branched saturated aliphatic hydrocarbon group of which carbon number is not less than 1 and not more than 6. The group is exemplified by a methyl group, an ethyl group, an n-propyl group, an isopropyl group, an n-butyl group, an isobutyl group, a t-butyl group, an n-pentyl group and an n-hexyl group. The group is preferably a C₁₋₄ alkyl group, more preferably a C₁₋₂ alkyl group, and most preferably a methyl group.

The term " C₂₋₆ alkenyl group" is a linear or branched unsaturated aliphatic hydrocarbon group of which carbon number is not less than 2 and not more than 6 and which has at least one carbon-carbon double bond. The group is exemplified by an ethenyl group, a 1-propenyl group, a 2-propenyl group, i.e. an allyl group, an isopropenyl group, a 2-butenyl group, a 3-butenyl group, an isobutenyl group, a pentenyl group and a hexenyl group. The group is preferably a C₂₋₄ alkenyl group, and more preferably a 2-propenyl group, i.e. an allyl group.

The term "C₂₋₆ alkynyl group" is a linear or branched unsaturated aliphatic hydrocarbon group of which carbon number is not less than 2 and not more than 6 and which has at least one carbon-carbon triple bond. The group is exemplified by an ethynyl group, a 1-propynyl group, a 2-propynyl group, a 2-butynyl group, a 3-butynyl group, a pentynyl group and a hexynyl group. The group is preferably a C₂₋₄ alkynyl group, and more preferably a C₂₋₃ alkynyl group.

The "amino group" is exemplified by a -NH₂ group, a mono(C₁₋₆ alkyl)amino group and a di(C₁₋₆ alkyl)amino group. More specifically, a mono(C₁₋₆ alkyl)amino group is exemplified by a methylamino group, an ethylamino group, an isopropylamino group, a t-butylamino group, an n-pentylamino group and an n-hexylamino group. Two C₁₋₆ alkyl groups in a di(C₁₋₆ alkyl) amino group may be the same or different from each other. A di(C₁₋₆ alkyl) amino group is exemplified by a dimethylamino group, a diethylamino group, a diisopropylamino group, a t-butylmethylamino group, a t-butylethylamino group, a di(t-butyl)amino group, a di(n-pentyl)amino group and a di(n-hexyl)amino group.

The "hydroxyamino group" is exemplified by -NHOH group and an N-(C₁₋₆ alkyl) hydroxyamino group. More specifically, an N-(C₁₋₆ alkyl) hydroxyamino group is exemplified by an N-methylhydroxyamino group, an N-ethylhydroxyamino group, an N-isopropylhydroxyamino group, an N-t-butylhydroxyamino group, an N-n-pentylhydroxyamino group and an N-n-hexylhydroxyamino group.

The "C₁₋₆ alkoxylamino group" is exemplified by an -N(C₁₋₆ alkoxy)H group and a (C₁₋₆ alkyl) (C₁₋₆ alkoxy)amino group. More specifically, an -N(C₁₋₆ alkoxy)H group is exemplified by a methoxyamino group, an ethoxyamino group, an isopropoxyamino group and a t-butoxyamino group. A (C₁₋₆ alkyl) (C₁₋₆ alkoxy) amino group is exemplified by a methoxymethylamino group, a methoxyethylamino group, an ethoxymethylamino group, an ethoxyethylamino group, an isopropoxymethylamino group and a t-butoxymethylamino group.

The term "C₁₋₆ alkoxy group" is a C₁₋₆ alkyloxy group. The group is exemplified by a methoxy group, an ethoxy group, an n-propoxy group, an isopropoxy group, an n-butoxy group, an isobutoxy group, a t-butoxy group, an n-pentoxy group and an n-hexoxy group. The group is preferably a C₁₋₄ alkoxy group, more preferably a C₁₋₂ alkoxy group, and even more preferably a methoxy group.

The term "(C₁₋₆ alkoxy) carbonyl group" is a C₁₋₆ alkyloxycarbonyl group. The group is exemplified by a methoxycarbonyl group, an ethoxycarbonyl group, an n-propoxycarbonyl group, an isopropoxycarbonyl group, an n-butoxycarbonyl group, an isobutoxycarbonyl group, a t-butoxycarbonyl group, an n-pentoxycarbonyl group and an n-hexoxyphenyl carbonyl. The group is preferably a (C₁₋₄ alkoxy) carbonyl group, more preferably a (C₁₋₂ alkoxy) carbonyl group, and even more preferably a methoxycarbonyl group.

The term "C₁₋₇ alkanoyl group" is a formyl group and a C₁₋₆ alkylcarbonyl group. The group is exemplified by a formyl group, an acetyl group, a propionyl group, i.e. an ethylcarbonyl group, a butyryl group, an isobutyryl group, i.e. an isopropylcarbonyl group, a pivaloyl group, a valeryl group, an isovaleryl group and a hexanoyl group. The group is preferably a C₁₋₄ alkanoyl group, more preferably C₁₋₃ alkanoyl group, and even more preferably an acetyl group.

The "tri(C₁₋₆ alkyl)silyl group" is exemplified by a trimethylsilyl group, a triethylsilyl group, a triisopropylsilyl group, a dimethylisopropylsilyl group, a diethylisopropylsilyl group and a t-butyldimethylsilyl group. The group is preferably a tri (C₁₋₄ alkyl)silyl group, and more preferably tri(C₁₋₂ alkyl)silyl group.

The term "halogen atom" contains a fluorine atom, a chlorine atom, a bromine atom and an iodine atom, and is preferably a chlorine atom or a bromine atom.

When a phenyl group of R² has substituent α, the number of the substituent is not less than 1 and not more than 5, preferably not more than 4, more preferably not more than 3, and even more preferably not more than 2.

When an alkyl group of R³ has substituent β, the number of the substituent depends on the number of the carbon atom of the alkyl group and the kind of the substituent β. In general, the number is not less than 1 and not more than 5, preferably not more than 4, more preferably not more than 3, and even more preferably not more than 2. The R³ is preferably a benzyloxy (C₁₋₆ alkyl) group, more preferably a benzyloxy (C₁₋₄ alkyl) group, and even more preferably a benzyloxy (C₁₋₂ alkyl) group.

When the number of the above substituent is not less than 2, the substituents may be the same or different from each other.

The benzylamine derivative (I) of the present invention may have one or more chiral centers in some cases. In such a case, there is an optical isomer. The range of the present invention includes both of the mixture thereof and individual isomer which is separated.

The benzylamine derivative (I) of the present invention may be a salt by an ordinary method. A salt of the benzylamine derivative (I) is preferably a publicly-known non-toxic pharmaceutically acceptable salt, and exemplified by an inorganic acid salt such as a hydrochloride, a hydrobromide, a sulfate and a phosphate; an organic acid salt such as an acetate, a malonate, a tartrate, a maleate, a methanesulfonate, a benzenesulfonate, a formate, a toluenesulfonate and a trifluoroacetate; an amino acid salt such as an alginate, an aspartate and a glutamate; an alkali metal salt such as a sodium salt and a potassium salt; an alkaline earth metal salt such as a calcium salt and a magnesium salt; and an organic base salt such as an ammonium salt, a trimethylamine salt, a triethylamine salt, a pyridine salt, a picoline salt and a dicyclohexylamine salt.

As the benzylamine derivative (I) of the present invention, the following compounds are preferably exemplified:

The benzylamine derivative, wherein n is not less than 2;

The benzylamine derivative, wherein X is a carbonyl group;

The benzylamine derivative, wherein R² is a C₂₋₆ alkenyl group;

The benzylamine derivative, wherein R³ is a benzyloxymethyl group;

The benzylamine derivative, wherein Y is a carbonyl group and R⁴ is a hydrogen atom.

The drug and the blood cancer drug of the present invention are characterized in comprising the above-described benzylamine derivative (I) or pharmaceutically acceptable salt thereof.

The benzylamine derivative (I) of the present invention is an active component of the above-described drug and blood cancer drug, and used for the treatment of blood cancer.

The method for treating blood cancer according to the present invention is characterized in comprising the step of administering the above-described benzylamine derivative (I) or pharmaceutically acceptable salt thereof.

### EFFECT OF THE INVENTION

The benzylamine derivative (I) of the present invention exhibits selective cytotoxicity against a blood cancer cell, particularly a multiple myeloma cell. More specifically, the benzylamine derivative does not exhibit cytotoxicity against not only a general epithelial cancer cell but also a normal cell. In addition, the benzylamine derivative of the present invention is also effective against an SP cell, i.e. Side Population Cell, which is a cause of recurrence of cancer. Therefore, the benzylamine derivative of the present invention is very useful as a safer drug and a safer blood cancer drug in contrast to a conventional anticancer drug of which selectivity is low and which is also toxic to a normal cell.

### BRIEF DESCRIPTION OF THE DRAWING

Figure 1 is a graph which demonstrates an anticancer activity against a human multiple myeloma cell of the benzylamine derivative according to the present invention and melphalan, which is generally used as a drug for treating multiple myeloma.
Figure 2 is a graph which demonstrates cytotoxicity of the benzylamine derivative according to the present invention against various kinds of cancer cells and a normal cell.
Figure 3(1) is a photograph of human multiple myeloma cells which were treated with the benzylamine derivative of the present invention and which were stained using Hoechst 33258, which is a nucleus staining agent; Figure 3(2) is a photograph of the cells which were stained using Annexin V, which is a marker of apoptosis; and Figure 3(3) is the analysis result of the cells using flow cytometry.
Figures 4 are the results of flow cytometry (1) in case where human multiple myeloma cells were treated with only a solvent as a negative control, (2) in case where the cells were treated with melphalan as a positive control, and (3) in case where the cells were treated with the benzylamine derivative of the present invention.
Figures 5 are the photographs which demonstrate the formation state of colonies in a methylcellulose culture medium (1) in case where human multiple myeloma cells were treated with only a solvent as a negative control, and (2) in case where the cells were treated with the benzylamine derivative of the present invention.
Figure 6 is a graph which demonstrates anticancer activities of the benzylamine derivative according to the present invention against a human multiple myeloma cell, a human acute myeloid leukemia cell and a human premyelocytic leukemia cell.
Figure 7 is a graph which demonstrates anticancer activities of melphalan, which is generally used as a drug for treating multiple myeloma, against a human multiple myeloma cell, a human acute myeloid leukemia cell and a human premyelocytic leukemia cell.

### MODE FOR CARRYING OUT THE INVENTION

For example, the benzylamine derivative of the present invention can be synthesized by the following scheme.

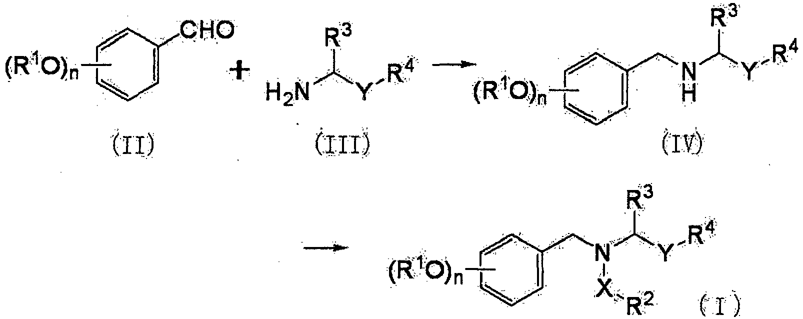

In the above scheme, the groups and the like are the same as the above.

Hereinafter, each step is described. If the compound has a reactive group which inhibits the reaction, the group may be protected and deprotected on a timely basis. For the kind of a protective group and a reaction condition in such a protecting reaction and deprotecting reaction, "PROTECTIVE GROUPS IN ORGANIC SYNTHESIS Second Edition" T. W. Green and P. G. M. Wuts, John Wiley & Sons, INC. , of which content is hereby incorporated by reference, may be referred.

First, the compound (IV) is synthesized by reacting the benzaldehyde compound (II) with the amine compound (III). The reaction is known as reductive amination.

The benzaldehyde compound (II) among the raw material compounds has a simple chemical structure. Therefore, the benzaldehyde compound may be purchased, if the compound is commercially available. Alternatively, the person skilled in the art can easily synthesize the benzaldehyde compound from a commercially available compound by an ordinary method. The amine compound (III) can be synthesized from a serine derivative or the like by the method described in Hart, S. A. et al., Org. Lett, volume 3, pp.1789-1791 (2001), the content of which is hereby incorporated by reference, and a similar method thereto.

For example, the above reaction can be carried out by dissolving the amine compound (III) in a solvent, adding the benzaldehyde compound (II) to the solution to obtain a Schiff base, and further adding a reducing agent thereto.

A solvent used in the above reaction is not particularly limited as long as the solvent can appropriately dissolve the raw material compounds and does not inhibit the reaction, and is exemplified by a halogenated hydrocarbon solvent such as dichloromethane, 1,2-dichloroethane and chloroform; an alcohol solvent such as methanol and ethanol; an ether solvent such as diethyl ether and tetrahydrofuran; and an aromatic hydrocarbon solvent such as benzene and toluene; and an amide solvent such as dimethylformamide and dimethylacetamide.

A concentration of the amine compound (III) may be appropriately adjusted, for example, to be not less than about 1 mg/mL and not more than about 20 mg/mL.

The benzaldehyde compound (II) and the amine compound (II) may be theoretically used in an equal molar. However, when one compound is more inexpensive or more available than the other compound, one compound may be excessively used in not less than about 1.01 times by mole and not more than about 1.2 times by mole relative to the other compound.

A temperature and a time of the reaction for obtaining the Schiff base may be appropriately adjusted, and the reaction may be carried out at not less than about 10°C and not more than about 50°C for not less than about 1 minute and not more than about 1 hour for speedy progress.

After the reaction to obtain the Schiff base, a reducing agent may be directly added to the reaction mixture. Such a reducing agent to be used is exemplified by sodium borohydride, lithium aluminum hydride, diisobutylaluminum hydride, sodium cyanoborohydride, lithium borohydride, hydrogenated triethylboron lithium, borane complex, triethylsilane, nickel borohydride and sodium triacetoxyborohydride. The reducing agent may be used in not less than about equal molar and not more than 1.2 times by mole relative to smaller one in molar between the benzaldehyde compound (II) and the amine compound (III).

When the reducing agent is added, a temperature of the reaction mixture is preferably once cooled down to 5°C or less using ice or the like. After the addition, the temperature may be gradually increased, and the reaction may be carried out at not less than about 10°C and not more than about 50°C for not less than about 5 hours and not more than about 50 hours.

After the reaction, a general aftertreatment may be carried out. For example, the solvent may be removed away, and then the compound (IV) may be purified with column chromatography, recrystallization or the like.

Next, -X-R² group is introduced in the compound (IV). As a reagent for introducing -X-R² group, an acid chloride, an acid bromide, an active ester and the like of the corresponding acid, i.e. HO-X-R², may be used. In the reaction, the compound (IV) and the above reagent may be reacted in the presence of a base.

A solvent used in the reaction is not particularly limited, and the same solvent used in the above-described reductive amination reaction may be used.

A reagent for introducing -X-R² group is preferably used in an excessive amount relative to the compound (IV). For example, the reagent may be used in not less than about 1.05 times by mole and not more than about 20 times by mole relative to the compound (IV).

As a base, an organic base is preferably used, since the reaction is carried out in an organic solvent. The base to be used is exemplified by triethylamine, pyridine, diazabicycloundecene, 1,8-bis(dimethylamino)naphthalene. An amount of the base to be used is at least equal molar to the reagent for introducing -X-R² group.

In the reaction, the above compounds may be mixed in a solvent. It is preferred that when the compounds are mixed, a temperature of the reaction mixture is cooled down to 5°C or less using ice or the like, since the reagent for introducing -X-R² group is highly reactive. After the compounds are mixed, the temperature may be gradually increased and the reaction may be carried out at not less than about 10°C and not more than about 50°C for not less than about 1 hour and not more than about 10 hours.

After the reaction, a general aftertreatment may be carried out. For example, the solvent may be removed away, and then the compound (IV) may be purified with column chromatography, recrystallization or the like.

In addition, the target benzylamine derivative can be synthesized by a functional group transformation reaction which is well-known to the person skilled in the art other than a deprotection reaction of a reactive group.

The benzylamine derivative (I) and pharmaceutically acceptable salt thereof according to the present invention can be used for treating blood cancer as a medicinal preparation containing the derivative or salt as an active component and a pharmaceutically acceptable organic or inorganic solid or liquid excipient which is suitable for oral administration or parenteral administration. Such a medicinal preparation can be developed in a form of an encapsulated formulation, a tablet, a sugar-coated tablet, a granule, an inhalant, a suppository, a solution, a lotion, a dispersion, an emulsion or the like. As necessary, a general additive agent, such as an auxiliary agent, a stabilizing agent, a wetting agent, an emulsifier and a buffering agent, can be added to the medicinal preparation.

A dosage amount of the benzylamine derivative (I) according to the present invention for a treatment is dependent on a severity, age, sex, condition or the like of a patient, and for example, a dosage amount of not less than about 0.001 mg/kg and not more than about 500 mg/kg on a basis of a patient's weight may be effective to treat blood cancer. In general, for example, the derivative can be administered in an amount of about 0.005 mg/kg and not more than about 1000 mg/kg on a basis of a patient's weight one day.

The present application claims the benefit of the priority date of Japanese patent application No. 2011-119668 filed on May 27, 2011, and all of the contents of the Japanese patent application No. 2011-119668 filed on May 27, 2011 are incorporated by reference.

### EXAMPLES

Hereinafter, the present invention is described in more detail with Examples. However, the present invention is not limited by the following Examples, and the present invention can be appropriately modified to be carried out within a range which adapts to the contents of this specification. Such a modified embodiment is also included in the range of the present invention.

### Example 1

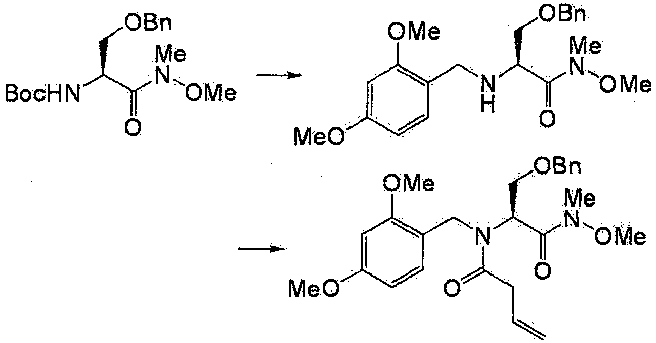

The above-described raw material compound was synthesized by the method described in Hart, S. A. et al., Org. Lett., volume 3, pp.1789-1791 (2001), the content of which is hereby incorporated by reference. To the raw material compound (2.00 g, 6.21 mmol), hydrogen chloride / ethyl acetate (4M, 30.0 mL) was added at 0°C. The mixture was stirred at the same temperature for 2 hours. Then, the solvent and the like were removed away under reduced pressure, and 1M aqueous solution of sodium hydroxide was added to the obtained residue. Then, extraction was carried out using chloroform. The organic layer was dried over anhydrous sodium sulfate and filtrated. The solvent was removed away under reduced pressure. The obtained residue (1.35 g, 5.67 mmol) was dissolved in 1,2-dichloroethane (182 mL), and 2,4-dimethoxybenzaldehyde (0.942 g, 5.67 mmol) was added thereto at room temperature. The mixture was stirred for 10 minutes. After the reaction mixture was cooled down to 0°C, sodium triacetoxyborohydride (1.20 g, 5.67 mmol) was added thereto. While the temperature was gradually increased to room temperature, the mixture was stirred for 1 day. After a saturated aqueous solution of sodium hydrogencarbonate was added to the reaction mixture, extraction was carried out using chloroform. The obtained organic layer was washed using saturated saline and dried over anhydride sodium sulfate. The mixture was filtered and the solvent was removed away under reduced pressure. The residue was purified by column chromatography using hexane / ethyl acetate = 1 / 4 as eluent, to obtain an yellow oily amine compound (1.77 g, 4.56 mmol) as a synthetic intermediate in a yield of 73%.

Separately, 3-butenoic acid (0.443 g, 5.15 mmol) was dissolved in methylene chloride (2.0 mL), and thionyl chloride (0.613 g, 5.15 mmol) was added at 0°C. The mixture was stirred at room temperature for 1 hour. The solvent and the like were removed away under reduced pressure. To the obtained residue, a methylene chloride solution (2.0 mL) of the above amine compound (0.200 g, 0.515 mmol) and triethylamine (0.718 mL, 5.15 mmol) were added at 0°C. While a temperature of the reaction mixture was gradually increased to room temperature, the reaction mixture was stirred for 3 hours. After a saturated aqueous solution of ammonium chloride was added to the reaction mixture, extraction was carried out with ethyl acetate. The obtained organic layer was successively washed with distilled water, a saturated aqueous solution of sodium hydrogencarbonate and saturated brine. Then, the organic layer was dried over anhydrous magnesium sulfate and filtered. The obtained residue was purified by column chromatography using hexane / ethyl acetate = 1 / 1 as eluent, to obtain the colorless oily target compound (0.172 g, 0.377 mmol) in a yield of 73%.

### Example 2

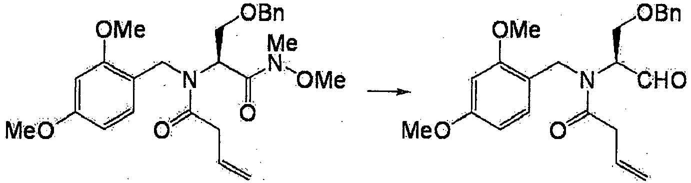

To a tetrahydrofuran solution (860 µL) of the compound obtained in Example 1 (40 mg, 88 µmol), lithium aluminum hydride (6.6 mg, 170 µmol) was added at -78°C. The mixture was stirred at the same temperature for 4 hours. Then, ethyl acetate and a saturated aqueous solution of ammonium chloride were added thereto at the same temperature. A temperature of the mixture was increased to room temperature, and a saturated aqueous solution of citric acid was further added thereto. The reaction mixture was extracted with ethyl acetate. The obtained organic layer was washed with saturated brine and dried over anhydrous sodium sulfate. The mixture was filtered, and then the solvent was removed away under reduced pressure. The obtained residue was purified by column chromatography using hexane / ethyl acetate = 2 / 1 as eluent, to obtain the yellow oily target compound (6.8 g, 17 µmol) in a yield of 20%.
¹H-NMR (CDCl₃, 400MHz) δ=3.30(1H, dd, J=16.8Hz and 6.8Hz), 3.37(1H, dd, J=16.8Hz and 6.8Hz), 3.78-3.90(1H, m), 3.79(3H, s), 3.81(3H, s), 4.11(1H, dd, J=9.6Hz and 4.0Hz), 4.42-4.60(2H, m), 4.46(1H, d, J=12.4Hz), 4.50(1H, d, J=12.4Hz), 4.73(1H, d, J=16.0Hz), 5.16(1H, dd, J=16.8Hz and 1.2Hz), 5.19(1H, dd, J=8.8Hz and 1.2Hz), 5. 97 (1H, ddt, J=16.8Hz, 8.8Hz and 6.8Hz), 6.42-6.47(2H, m), 7.12(H, d, J=8.8Hz), 7.24-7.34(5H, m), 9.29(1H, s)

### Example 3: Anticancer activity against multiple myeloma cell

Human multiple myeloma cell line RPMI8226 was seeded on a 96 well plate in a density of 1×10⁴ cells/well, and incubated under 5% carbon dioxide atmosphere at 37°C overnight. Separately, the compound obtained in Example 2 was dissolved in dimethylsulfoxide, and added to each well so that the final concentration of the compound became 0.5 µM, 1 µM, 2 µM, 3 µM, 12.5 µM, 25 µM or 50 µM.

In addition, melphalan, which is a multiple myeloma therapeutic agent, was added to each well so that the final concentration of the compound became 10 µM, 20 µM, 30 µM, 40 µM or 50 µM as positive controls for comparison. Further, an experimental case in which only dimethylsulfoxide was added in the final concentration of 0.5 v/v% without adding the compound was provided.

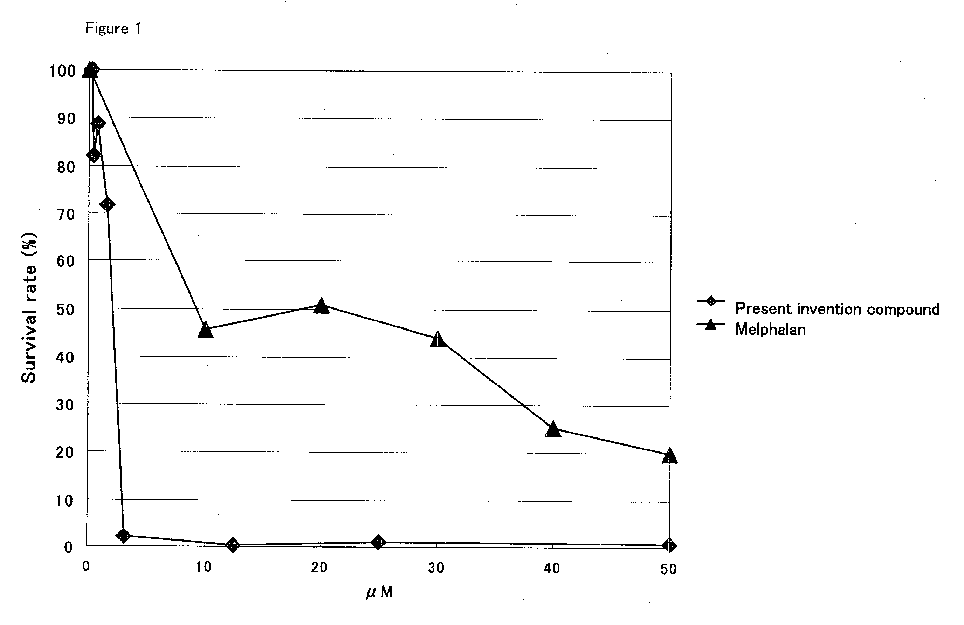

Then, after the cell was cultured under 5% carbon dioxide atmosphere at 37°C for 3 days, an absorbance of formazan, which is an orange pigment produced by a living cell, at the wavelength of 420 nm was measured using a reagent for measuring cell proliferation/cytotoxicity (manufactured by Dojin Chemical Co., Cell Counting Kit-8). The absorbance value in the case where the present invention compound or melphalan was not added was set to be 100, and relative absorbance values relative to the value are demonstrated as Figure 1. In addition, IC₅₀ values were calculated from the obtained results.

As shown in Figure 1, it was confirmed that the present invention compound has an anticancer activity against a multiple myeloma cell in a much lower concentration than melphalan, which is generally used for the treatment of multiple myeloma. In addition, the present invention compound exhibited anticancer activity in about one-twentieth concentration relative to melphalan, as the IC₅₀ value of the present invention compound against a multiple myeloma cell was 1.12 µM, whereas the IC₅₀ value of melphalan was 21.0 µM.

### Example 4: Test for selectivity

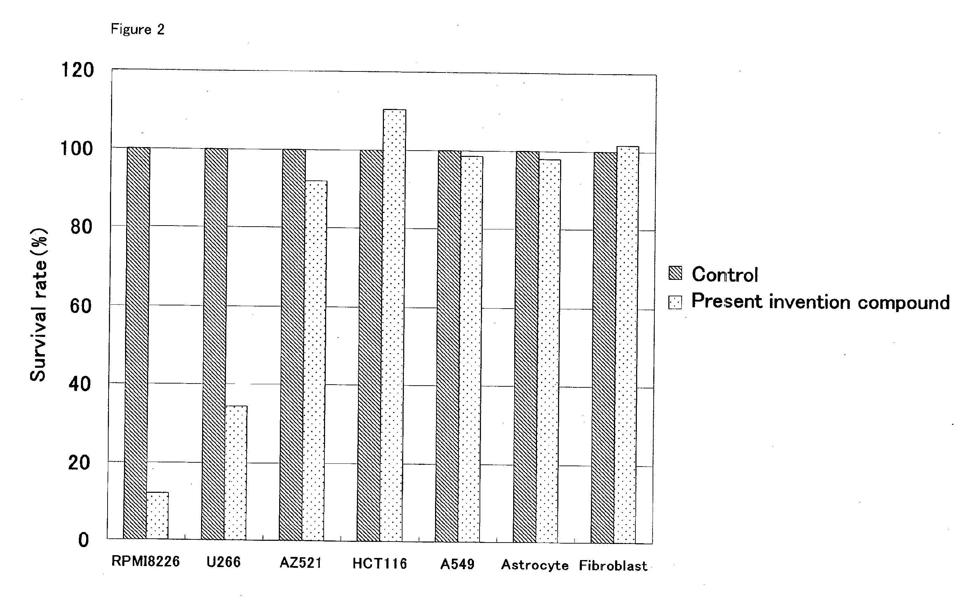

Cytotoxicity against each cell was measured similarly to Example 3 except that human multiple myeloma cell lines RPMI8226 and U266, human stomach cancer cell line AZ521, human colorectal cancer cell line HCT116, human lung cancer cell line A549, healthy astrocyte derived from mouse brain and healthy fibroblast derived from human skin were used as tested cells, and the final concentration of the present invention compound at each well was set to be 5 µM. The absorbance value when the compound was not added was set to be 100, and the resultant relative absorbance values relative to the value are demonstrated as Figure 2.

As shown in Figure 2, the present invention compound does not exhibit cytotoxicity against not only a healthy cell but also a general cancer cell such as a stomach cancer cell, a colorectal cancer cell and a lung cancer cell; on the other hand, the present invention compound exhibits clear cytotoxicity against a multiple myeloma cell, which is a kind of a blood cancer cell. From the result, it was demonstrated that the present invention compound is very useful as a drug for treating blood cancer, since the compound exhibits selective cell cytotoxicity.

### Example 5: Test for apoptosis activity

It was examined whether cell death due to the present invention compound is apoptosis or not.

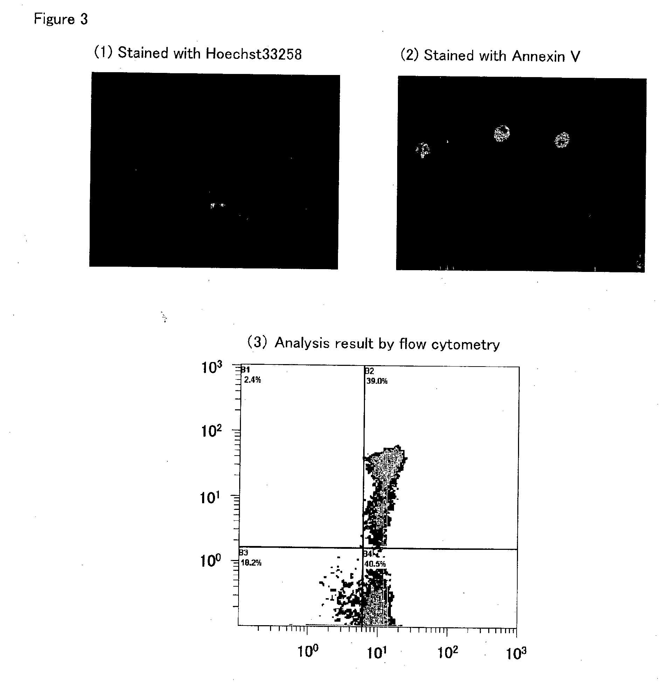

Specifically, human multiple myeloma cell line RPMI8226 was incubated similarly to Example 3 except that the final concentration of the present invention compound was set to be 5 µM. After 24 hours, the cell was recovered and stained using Hoechst33258, which is a nucleus staining agent, to take fluorescence photographs using a fluorescence microscope manufactured by zeiss. Separately, the cell was stained using Annexin V and propidium iodide, which are markers of apoptosis, using Annexin V dyeing kit manufactured by Roche, and then fluorescence photographs were similarly taken. In addition, the cell was analyzed using a flow cytometer ("EPICS ALTRA" manufactured by Beckman Coulter, Inc.). The microscope photograph of the cells stained with Hoechst33258 is shown as Figure 3(1), the microscope photograph of the cells stained with Annexin V and propidium iodide is shown as Figure 3(2), and the result of flow cytometry analysis of the cells stained with Annexin V and propidium iodide is shown as Figure 3(3).

As Figure 3(1), when a human multiple myeloma cell was treated with the present invention compound, Hoechst33258 was remarkably ingested in the cell and the ingested Hoechst33258 yielded fluorescence. It is considered that this result is because chromatin aggregated in cell nucleus of apoptotic cell and Hoechst33258 was bound to the aggregated chromatin. In addition, as Figure 3(2), when a human multiple myeloma cell was treated with the present invention compound, the cell was also positive for Annexin V, which is a marker of apoptosis. Furthermore, as Figure 3(3), when a human multiple myeloma cell was treated with the present invention compound, early apoptosis and late apoptosis were caused. In early apoptosis, a cell is negative for propidium iodide and positive for Annexin V; and in late apoptosis, a cell is positive for propidium iodide and positive for Annexin V. From the above result, it was experimentally demonstrated that the present invention compound causes apoptosis of a multiple myeloma cell.

### Example 6: Anticancer activity against SP cell

A stem cell can undergo differentiation into various tissues and cells such as a hematopoietic stem cell, and has the quality to exclude fluorescent chemical substance such as Hoechst33258 extracellularly. Therefore, when a stem cell is stained with such a fluorescent chemical substance, then the cell is analyzed by flow cytometry and the results at the wave-lengths of 450 nm and 675 nm are deployed on a two-dimensional plane, the cell is found in a region which demonstrates low stainability. A cell having such properties is called as an SP cell, i.e. Side Population Cell. An SP cell is included in cancer cell group, and it is known that an SP cell exhibits drug resistance and provides cause of recurrence. Therefore, an effect of the present invention compound against a cancerous SP cell was examined.

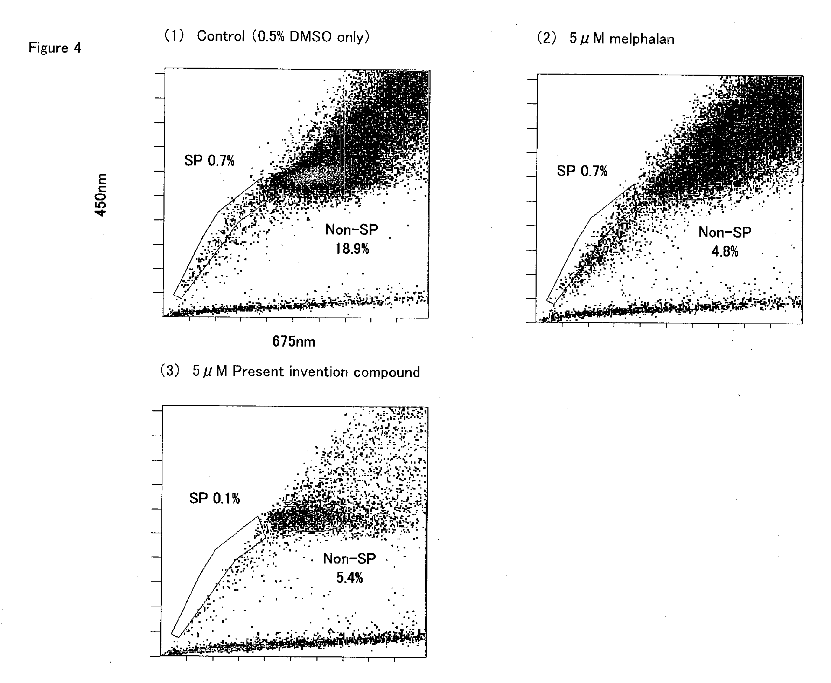

Specifically, human multiple myeloma cell line RPMI8226 was incubated in the presence of the present invention compound similarly to Example 4. The cell was dispersed in 3% FBS DMEM so that the concentration became 1×10⁶ cells/mL. A temperature of the dispersion was increased to 37°C, and then Hoechst33342 was added thereto so that the concentration thereof became 5 µg/mL. The mixture was incubated at 37°C for 60 minutes under a shaded state with stirring every 20 minutes. Then, the mixture was centrifuged at 800xg for 3 minutes, and the supernatant was removed. Further, a washing procedure in which cooled 2% FBS PBS was added, the mixture was centrifuged and the supernatant was removed was repeated two times. Separately, propidium iodide was dissolved in PBS to obtain a 200 µg/mL solution. The washed cell was dispersed again in the solution in the concentration of about 5×10⁶ cells/mL. The cell was exposed to a laser beam of 365 nm using a flow cytometer ("EPICS ALTRA" manufactured by Beckman Coulter, Inc.). The emitted fluorescence was passed through a bandpass filters of 450 nm and 675 nm, and deployed to obtain a two-dimensional dot plot. In addition, similar experiments were carried out with respect to the cases where dimethylsulfoxide only was added in the final concentration of 0. 5 v/v% without the compound as a negative control and melphalan was added in the same final concentration, 5 µM, as a positive control. The result of the negative control is demonstrated as Figure 4(1), the result of the positive control is demonstrated as Figure 4(2), and the result obtained by using the present invention is demonstrated as Figure 4(3). In addition, the ratios of stained cells in SP region and non-SP region relative to the total cells were calculated from the result of flow cytometry and are demonstrated in Table 1.

**Table 1**

| | SP region | Non-SP region |
|---|---|---|
| Control (0.5%DMSO only) | 0.680±0.0173% | 19.2±0.295% |
| 5 *µ*M M elphalan | 0.563±0.0777% | 5.05±0.414% |
| 5 *µ*M P resent invention compound | 0.163±0.0503% | 5.36±0.235% |

From the above-described result, it was confirmed that when a human multiple myeloma cell was treated with dimethylsulfoxide only, SP cells were present in a region in which the radiances at both wave-lengths were low. In addition, when the cell was treated with melphalan, the ratio of SP cells was decreased in some degree compared to control, but SP cells were sufficiently present. Melphalan is used for treating multiple myeloma, but in such a case, cure is difficult and prognosis is less favorable. It is thought that this is because SP cells remain when melphalan is used for the treatment and the remaining cancerous SP cells regrow and spread. On the other hand, when the cell was treated with the present invention compound, the ratio of stained cells relative to the whole cells in SP region was decreased to 0.163±0.0503% in comparison with 0.680±0.0173% of the control. Therefore, it can be thought that the present invention compound can not only cause apoptosis of a multiple myeloma cell but also inhibit SP cell thereof; as a result, recurrence can be suppressed.

### Example 7

A colony forming assay was carried out using a methylcellulose culture medium as an experiment having high correlativity to a tumor forming experiment using a mouse or the like.

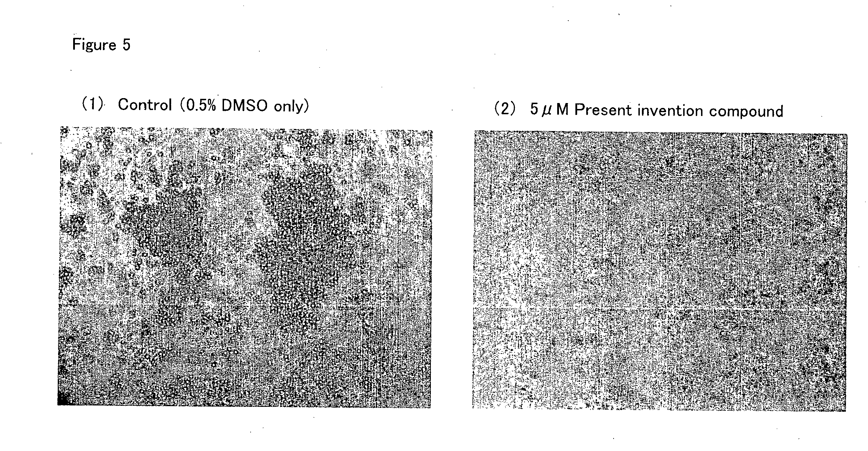

Specifically, human multiple myeloma cell line RPMI8226 was incubated similarly to Example 3 except that the final concentration of the present invention compound was set to be 5 µM. After 24 hours, the cell was recovered. The number of the cell was measured, and then 1×10⁵ cells and 1 mL of a methylcellulose culture medium manufactured by STEMCELL Technologies were mixed. The mixture was mixed using a rotator at 37°C for 1 hour, and then total amount of the mixture was added on 6 well plate. The cell was incubated under 5% carbon dioxide atmosphere at 37°C for 14 days, and then a photograph was taken using a microscope manufactured by zeiss. In addition, similar experiment was carried out using a culture medium to which 0.5% of dimethylsulfoxide was added as control for comparison. The result of the control is demonstrated as Figure 5 (1) and the result of the case where the present invention compound was used is demonstrated as Figure 5(2).

As Figure 5, colonies of multiple myeloma cells were formed in control; on the other hand, in a culture medium containing the present invention, no colony was observed and the formation of colony was remarkably inhibited. Therefore, it can be thought that even if a multiple myeloma cell infiltrates, a tumor hardly forms; as a result, reoccurrence also hardly causes.

### Example 8

The effect of the present invention compound against blood cancer cells other than a human multiple myeloma cell was examined.

Human acute myelogenous leukemia cell line KG1 and human promyelocytic leukemia cell line HL60 in addition to human multiple myeloma cell line RPMI8226 were seeded in a highly nutrient medium containing 10% FBS (RPMI1640, 100 µL) in a density of 1×10³ cells/well. The cells were incubated under 5% carbon dioxide atmosphere at 37°C overnight. Separately, the compound of Example 2 was dissolved in DMSO and the obtained solution was diluted with the same culture medium. The mixture was added to each well so that the final concentrations of the compound became 0.39 µM, 0.78 µM, 1.56 µM, 3.13 µM, 6.25 µM, 12.5 µM or 50 µM.

In addition, melphalan, which is a multiple myeloma therapeutic agent, was added to each well at the same final concentrations as positive controls for comparison. Further, an experimental case in which only dimethylsulfoxide was added in the final concentration of 0.2 to 0.8 v/v% without adding the compound was provided.

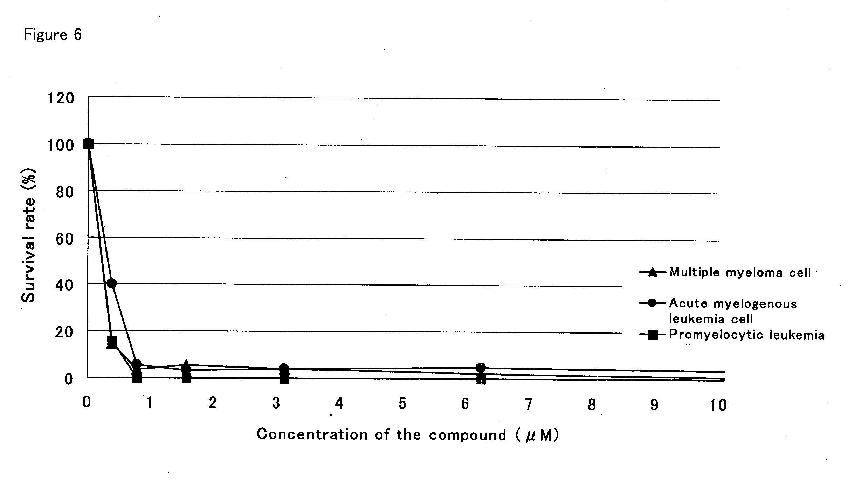

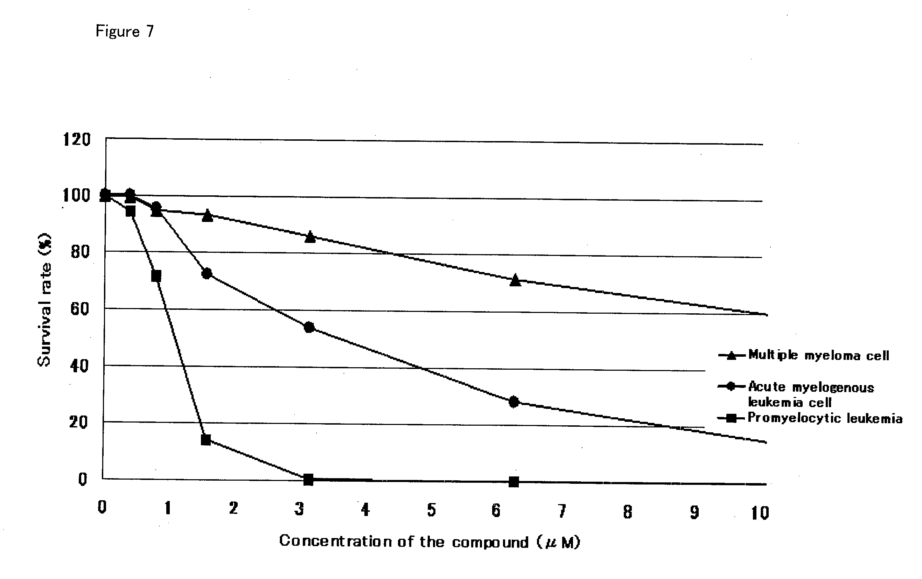

Then, after the cells were cultured under 5% carbon dioxide atmosphere at 37°C for 72 hours, the culture medium was removed and a culture medium obtained by mixing 100 µL of the above culture medium and 10 µL of WST-8, which is a reagent for measuring a cell number, was added to each well. After the cells were cultured under 5% carbon dioxide atmosphere at 37°C for 2 hours, an absorbance at 450 nm was measured. The absorbance value when the cells were treated with DMSO only was set to be 100%, and a ratio of absorbance values at each concentration of the present invention compound or melphalan relative to the value were calculated as survival rate of each blood cancer cell. In addition, EC₅₀, i.e. 50% effective concentration value, of each compound against each blood cancer cell was calculated from the obtained results. The survival rate results of the present invention compound are demonstrated as Figure 6, and the survival rate results of melphalan are demonstrated as Figure 7. In addition, the results of EC₅₀, i.e. 50% effective concentration value, are demonstrated in Table 2.

**Table 2**

| | Multiple myeloma cell | Acute myelogenous leukemia cell | Promyelocytic leukemia cell |
|---|---|---|---|
| Present invention compound | 64nM | 303nM | 300nM or less (measurement limit or less) |
| M elphalan | 9.82 *µ*M | 3.69 *µ*M | 734m M |

As shown in Figures 6 and 7, it was experimentally demonstrated that the compound of the present invention exhibits an anticancer activity against an acute myelogenous leukemia cell and a promyelocytic leukemia cell in addition to a multiple myeloma cell in much lower concentration than melphalan, which is generally used as a multiple myeloma therapeutic agent.

## Claims

1. A benzylamine derivative represented by the following formula (I) or a pharmaceutically acceptable salt thereof: 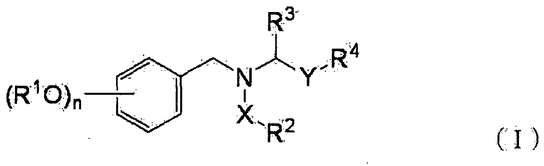 wherein
X is a carbonyl group, an oxycarbonyl group, a sulfoxide group or a sulfonyl group;
Y is a carbonyl group or -CH(OR⁵)-;
R¹ is a hydrogen atom, a C₁₋₆ alkyl group or a benzyl group; R² is a C₁₋₆ alkyl group, a C₂₋₆ alkenyl group, a C₂₋₆ alkynyl group or a phenyl group optionally having a substituent α;
R³ is a C₁₋₆ alkyl group optionally having a substituent β; R⁴ is a hydrogen atom, an amino group, a hydroxyamino group, a C₁₋₆ alkoxyamino group, a carboxy group or a (C₁₋₆ alkoxy) carbonyl group;
R⁵ is a hydrogen atom, a C₁₋₇ alkanoyl group or a tri (C₁₋₆ alkyl)silyl group;
substituent α is one or more substituents selected from the group consisting of a C₁₋₆ alkyl group, a C₁₋₆ alkoxy group, a halogen atom, a cyano group and a nitro group;
substituent β is one or more substituents selected from the group consisting of a halogen atom, a hydroxy group, a C₁₋₆ alkoxy group and a benzyloxy group;
n is an integer of not less than 1 and not more than 5.

2. The benzylamine derivative according to claim 1, wherein R¹ is a C₁₋₆ alkyl group.

3. The benzylamine derivative according to claim 1 or 2, wherein n is not less than 2.

4. The benzylamine derivative according to any one of claims 1 to 3, wherein X is a carbonyl group.

5. The benzylamine derivative according to any one of claims 1 to 4, wherein R² is a C₂₋₆ alkenyl group.

6. The benzylamine derivative according to any one of claims 1 to 5, wherein R³ is a benzyloxymethyl group.

7. The benzylamine derivative according to any one of claims 1 to 6, wherein Y is a carbonyl group and R⁴ is a hydrogen atom.

8. A drug, comprising the benzylamine derivative or pharmaceutically acceptable salt thereof according to any one of claims 1 to 7.

9. A blood cancer drug, comprising the benzylamine derivative or pharmaceutically acceptable salt thereof according to any one of claims 1 to 7.

10. A benzylamine derivative represented by the following formula (I) or a pharmaceutically acceptable salt thereof for use in the treatment of blood cancer: 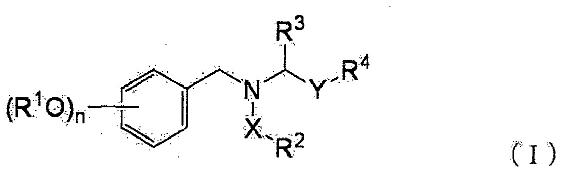 wherein
X is a carbonyl group, an oxycarbonyl group, a sulfoxide group or a sulfonyl group;
Y is a carbonyl group or -CH(OR⁵)-;
R¹ is a hydrogen atom, a C₁₋₆ alkyl group or a benzyl group; R² is a C₁₋₆ alkyl group, a C₂₋₆ alkenyl group, a C₂₋₆ alkynyl group or a phenyl group optionally having a substituent α;
R³ is a C₁₋₆ alkyl group optionally having a substituent β;
R⁴ is a hydrogen atom, an amino group, a hydroxyamino group, a C₁₋₆ alkoxyamino group, a carboxy group or a (C₁₋₆ alkoxy) carbonyl group;
R⁵ is a hydrogen atom, a C₁₋₇ alkanoyl group or a tri (C₁₋₆ alkyl)silyl group;
substituent α is one or more substituents selected from the group consisting of a C₁₋₆ alkyl group, a C₁₋₆ alkoxy group, a halogen atom, a cyano group and a nitro group;
substituent β is one or more substituents selected from the group consisting of a halogen atom, a hydroxy group, a C₁₋₆ alkoxy group and a benzyloxy group;
n is an integer of not less than 1 and not more than 5.

11. A method for treating blood cancer, comprising the step of administering a benzylamine derivative represented by the following formula (I) or a pharmaceutically acceptable salt thereof: 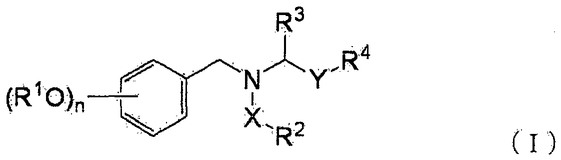 wherein
X is a carbonyl group, an oxycarbonyl group, a sulfoxide group or a sulfonyl group;
Y is a carbonyl group or -CH(OR⁵)-;
R¹ is a hydrogen atom, a C₁₋₆ alkyl group or a benzyl group; R² is a C₁₋₆ alkyl group, a C₂₋₆ alkenyl group, a C₂₋₆ alkynyl group or a phenyl group optionally having a substituent α;
R³ is a C₁₋₆ alkyl group optionally having a substituent β;
R⁴ is a hydrogen atom, an amino group, a hydroxyamino group, a C₁₋₆ alkoxyamino group, a carboxy group or a (C₁₋₆ alkoxy) carbonyl group;
R⁵ is a hydrogen atom, a C₁₋₇ alkanoyl group or a tri (C₁₋₆ alkyl)silyl group;
substituent α is one or more substituents selected from the group consisting of a C₁₋₆ alkyl group, a C₁₋₆ alkoxy group, a halogen atom, a cyano group and a nitro group;
substituent β is one or more substituents selected from the group consisting of a halogen atom, a hydroxy group, a C₁₋₆ alkoxy group and a benzyloxy group;
n is an integer of not less than 1 and not more than 5.
